# EUROPEAN PATENT APPLICATION

(11) **EP 2 039 767 A1**
(43) Date of publication of application: **25.03.2009**
(21) Application number: 07018695.2
(22) Date of filing: 24.09.2007
(51) Int. Cl.: C12N 15/11, A61K 31/7125

(54) **PNA conjugates targeted to the EMU enhancer of the IGH locus as therapeutic agent for BCL-2 translocation-driven follicular cell lymphoma clonal expansion**

(71) Applicant: Istituto nazionale per la ricerca sul cancro, 16132 Genova (IT)
(72) Inventor: Boffa, Lidia C., 16132 Genova (IT); Cutrona, Giovanna, 16132 Genova (IT); Ferrarini, Manlio, 16132 Genova (IT)
(74) Representative: Minoja, Fabrizio

(57) **Abstract**

The use of Peptide Nucleic Acids complementary to the Eµ enhancer of the IgH chain locus on chromosome 14 (PNAEµwt) for the preparation of a medicament for the treatment of Follicular Cell Lymphoma.

SEQUENCE LISTING

<110>  ISTITUTO NAZIONALE PER LA RICERCA SUL CANCRO

<120>  PNA CONJUGATES TARGETED TO THE EMU ENHANCER OF THE IGH LOCUS AS
       THERAPEUTIC AGENT FOR BCL-2 TRANSLOCATION DRIVEN FOLLICULAR CELL
       LYMPHOMA CLONAL EXPANSION

<130>  scb1896eur

<160>  4

<170>  PatentIn version 3.3

<210>  1
<211>  7
<212>  PRT
<213>  artificial peptide

<220>
<223>  artificial peptide

<400>  1

Pro Lys Lys Lys Arg Lys Val
1               5


<210>  2
<211>  18
<212>  DNA
<213>  artificial oligonucleotide

<220>
<223>  artificial oligonucleotide

<400>  2
gcaggaagca ggtcaccg                                                    18


<210>  3
<211>  18
<212>  DNA
<213>  artificial oligonucleotide

<220>
<223>  artificial oligonucleotide

<400>  3
gtaggaagta ggtcatcg                                                    18


<210>  4
<211>  16
<212>  DNA
<213>  artificial oligonucleotide

<220>
<223>  artificial oligonucleotide

<400>  4

ctacgagtgg gatgcg 16

## Description

The present invention refers to the use of Peptide Nucleic Acids (PNAs) complementary to the Eµ enhancer of the IgH chain locus on chromosome 14 (PNAEµwt) for the preparation of a medicament for the treatment of Follicular Cell Lymphoma.

### Background of the invention

Follicular Cell Lymphoma (FCL) is a non-Hodgkin's lymphoma (NHL) that exhibits marked differences in the incidence rates across geographic regions: extremely low incidence rates are reported among most Asian countries, and comparatively high rates are reported in North America and Western Europe.(1)

At present the standard FCL treatment consists of high dose conventional chemotherapy (CHOP: **C**ytoxan, **H**ydroxyrubicin (Adriamycin), **O**ncovin (Vincristine), **P**rednisone (Prednisone) or FCM: (**F**ludarabine, **C**yclophosphamide, and **M**itoxantrone) in combination with the monoclonal anti-CD20 antibody rituximab; (2).

Several second- line therapies exist for cases of FCL who relapse or fail first- line therapies. In some young patients, ASCT (Autologous Stem Cells Transplantation) following high dose chemotherapy is also performed.

Radioimmunotherapy (RIT) with 90Y-ibritumom ab [ibritumomab tiuxetan (Zevalin^{®})](3) has also been attempted in experimental clinical trials.

Recently enhanced efficacy of rituximab therapeutic properties if administered in synergy with an already clinically developed *bcl-2* antisense (G3139, Genasense; Genta, Inc. Berkeley Heights, NJ) has been demonstrated in SCID (Severely Compromised Immune Deficient) mouse/human FCL cell graft (4).

Despite the wide array of therapeutic strategies now available, patients with FCL stage III/IV tend to relapse repeatedly. Therefore the disease can not be considered as "cured" in any of these patients. In fact most of them succumb to FCL.

The t(14;18) chromosomal translocation is a hallmark of FCL. This cytogenetic abnormality is detected by FISH (Fluorescent In Situ Hybridization) and/or DNA analysis in more than 80% of FCL cases. In this translocation the *bcl-2* gene, normally located on chromosome 18, is juxtaposed with the potent Eµ enhancer of the immunoglobulin heavy chain gene(IgH) locus on chromosome 14 (5,6). This enhancer overrides *bcl-2* normal gene control elements and drives inappropriately high level of *bcl-2* expression (7,8). *Bcl-2* is an anti-apoptotic gene; therefore, *bcl-2* hyperexpression blocks apoptosis in follicular center cells which are transformed from short-lived cells into long-lived cells

We have conducted a series of preclinical/therapeutic studies on Burkitt's Lymphoma (BL) cells implementing a novel anti-gene approach utilizing PNAs. (10,11,12,13).

PNAs are nucleic acid analogs in which the natural backbone is substituted with uncharged pseudopeptidic 2-aminoethyl glycine units. (14) They are resistant to nucleases and proteases and bind with high affinity and specificity to complementary RNA and DNA. Moreover, PNAs can invade duplex DNA and hybridize with complementary sequences successfully displacing the natural cDNA strand.

Similar to FCL, c-myc expression in BL cells is generally upregulated as a consequence of its translocation from chromosome 8 to chromosome 14 where it is juxtaposed with the IgH chain locus encompassing the Eµ enhancer. (9)

We have previously shown, using the PNA anti-gene approach, that in BL (10)
- a specific PNA complementary to a unique sequence on the *c-myc* second exon (PNAmyc), when linked to the appropriate NLS, can specifically inhibit the expression of *c-myc*;
- a specific PNA complementary to the Eµ enhancer of the IgH chain locus on chromosome 14 (PNAEµwt) can cause a selective downregulation of the translocated c-myc (11).

We have recently reported that PNAEµ, if chronically administered to mice, can hinder the *in vivo* growth of tumors resulting from inoculation by BL cells. (13)

Furthermore, we have shown that all PNAEµ-NLS lack *in vivo* toxicity (13) and *in vitro* genotoxicity, mutagenicity, clastogenicity (15) and immunogenicity (16). Also, in the appropriate mouse model system, PNAEµ-NLS pharmacokinetics and lack of toxicity have been described.

### Brief Description of the Drawings

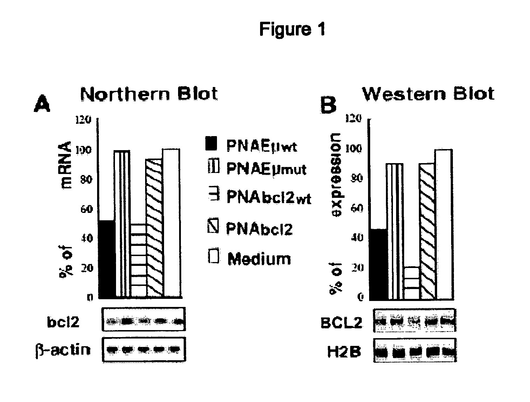

### Fig. 1. Assessment of bcl-2 down-regulation by PNAEµw in viable FCL cells in culture.

***A***, *bcl-2* mRNA expression. RNA was extracted from cells cultured for 18 h in the presence of the indicated PNAs and analyzed by Northern Blot with β*-actin* or *bcl-2* -specific probes. *bcl-2* mRNA concentrations were determined from the auto radiographic signals and expressed as percentage of mRNA of the unsusceptible β*-actin* gene.

***B*,** BCL2 protein expression. BCL2 protein was detected by Western blot in cells cultured for 48 h in the presence of the indicated PNAs. BCL2 concentrations are here normalized (NIH image) to histone H2B concentrations and reported as percentages. The data in *A* and *B* are representative of three experiments.

### Fig. 2. Growth inhibition of FCL cells by PNAEµwt

Growth curve of DOHH2 cells, plated at identical number (5 x 10⁵) and then treated with different PNAs (□, medium; ■, PNAEµwt;•, PNAEµmut;○, PNA*bcl*-2wt).

### Fig.3. FCL cell death induced by PNAEµwt is due to apoptosis.

FCL cells were incubated with the indicated PNAs for different times. Apoptotic cells and dead cells were detected, by flow cytometry, by Annexin-V binding and PI incorporation, respectively.

### Fig.4. FCL tumors growth in mice treated post-inoculum with PNAs.

Tumor volumes (in mm³) after inoculum of FCL cells in mice: untreated (-○-) or treated every other day (**^**) with 100 µl of 1 mM PNAEµwt (-■-) or PNAEµmut (...•...).

### Summary of the Invention

We have now found that PNAEµ specifically downregulates the hyperexpression of the *bcl-2* oncogene in FCL cells with consequent apoptosis induction and inhibition of malignant cell proliferation *in vitro* and tumor growth *in vivo.* In FCL, because of a t(18;14) translocation, the *bcl-2* oncogene becomes juxtaposed with the Eµ enhancer of the IgH locus in chromosome 14. Consequently the *bcl-2* gene becomes upregulated and hyperexpressed. The BCL2 protein, which has an anti-apoptotic effect by preventing mitochondrial damage, prevents follicular center cell apoptosis. The cells thus become immortalized and give rise to the expansion of an indolent proliferating clone.In previous studies, we demonstrated that PNAEµ can selectively inhibit the expression of translocated *c-myc* in BL. In this neoplasia, because of a t(8;14) translocation, *c-myc* is juxtaposed with the Eµ enhancer and upregulated. Because of the capacity of *c-myc* to drive the cells into the cell cycle, BL is characterized by a very active cell proliferation. Treatment with PNAEµ *in vitro* inhibits BL cells proliferation and induces some plasma cell differentiation.

We have now found that PNAEµwt is also able, by downregulating the antiapoptotic *bcl-2* gene, to induce the
- *In vitro* apoptotic death of FCL cells in culture and
- *In vivo* inhibition of tumor growth in SCID mice inoculated with human FCL cell lines.

The invention shows that FCL exposure in culture to PNAEµ, by blocking the target Enhancer, results in the inhibition not only of the expression of the *bcl-2* oncogene but also of its regulatory cellular function.

Furthermore, *in vivo* subcutaneous inoculation in SCID mice of FCL cells reproducibly induces tumors formation. (4) We have preliminary data that in this animal model, chronic intravenous administration of PNAEµwt to mice, already inoculated with FCL cells, selectively causes increased latency of tumor appearance and decreased final tumor size.

Apoptotic cell death and inhibition of tumor growth were specific since they were not observed with PNAEµmut-carrying sequence mutations. These data confirm the potential therapeutic value of PNA targeted to regulatory non-coding regions.

Design of a PNA-NLS conjugate, complemetary to the coding sequence of *bcl-2,* aimed to provide e direct selective gene-targeted therapy of FCL probably would not be applicable *in vivo.*

### Detailed description of the invention

### PNA-NLS design

PNAs were manually synthesized with monomers by the PerSeptive Biosystem^{™} using solid-phase peptide synthesis by Boc chemistry. The peptidic end (NLS: PKKKRKV) (SEQ ID NO: 1) was synthesized first, followed by the PNA sequence portion.

All compounds were purified by reverse-phase high-performance liquid chromatography on a Shimadzu LC-9 preparative high-performance chromatography system equipped with a Phenomenex C18 Luna column (21.20 IDx250mm length). Mass spectra of each compound were obtained in the positive ion mode using an ion trap mass spectrometer (MSD 1100 - Agilent Technologies, Palo Alto, CA, USA) equipped with an electro spray ion source. (13)

The PNA oligomers used were:
- PNAEµwt GCAGGAAGCAGGTCACCG-NLS [GenBank accession no.X00253. 1, bases136-157 (SEQ ID NO: 2)]
- PNAEµmut GTAGGAAGTAGGTCATCG-NLS (SEQ ID NO: 3).
- PNA**bcl2** CTACGAGTGGGATGCG-NLS [GenBank accession no.M13995, bases227-242 (SEQ ID NO: 4)]

### in vitro cell culture and treatments

### Cell culture.

The human FCL cell line DOHH2 (17) with a chromosomal translocation t(14;18)(q32;q21) was used and kept in culture in RPMI 1640 medium (Seromed-Biochrom KG, Berlin, D) supplemented with 10% fetal bovine serum (FBS) (Seromed), 1% glutamine, 1% Na pyruvate and 2% penicillin-streptomycin.

### Evaluation of Cell Growth, Cell Cycle, and Apoptosis.

The variations in the growth curves of cell lines treated with PNAs were determined by cell counts. (10,11) Cell cycle analysis was performed by PI staining in hypotonic solution and flow cytometry (FACStar; Becton-Dickinson, San Jose, CA;). Cell viability was evaluated by PI staining of intact cells in isotonic solution followed by flow cytometry. (10,11)

### Bcl-2 gene transcription.

Cellular levels of the *bcl-2* protein were determined by Western blot analysis as already reported(10,11) in FCL cells in culture with both PNAEµwt and PNAEµmut.

### Ability of PNAEµwt to inhibit bcl-2 transcription in viable FCL cells in culture.

We previously found that PNAmyc, when linked to NLS peptide, was able not only to enter BL cells but also BL nuclei. We investigated whether PNAEµwt, when linked to NLS peptide, was able to enter FCL cells in culture and inhibit the hyperexpression of the *bcl-2* oncogene.

DOHH2-FCL cells were incubated, at increasing exposure times, in the presence of the various test and control PNAs *in vitro* and their effect on both *bcl-2* mRNA transcription and *bcl-2* protein expression were evaluated.

As shown in Fig.1, *bcl-2* mRNA transcription (Fig1A) and *bcl-2* translation(Fig1B) in FCL cells at 48 hours of exposure, were inhibited not only by the antigene PNA***bcl-2*** targeted to the coding sequence of the protooncogene but also to a comparable extent by PNAEµwt targeted to the Eµ enhancer.

### Modulation of Proliferation and Apoptosis of DOHH2 Cells in culture by PNAEµwt.

Next, we tested whether the proliferation of DOHH2-FCL cells caused by *bcl-2* deregulation could be blocked not only by direct targeting of the *bcl-2* gene but also by targeting the Eµ with PNAEµwt.

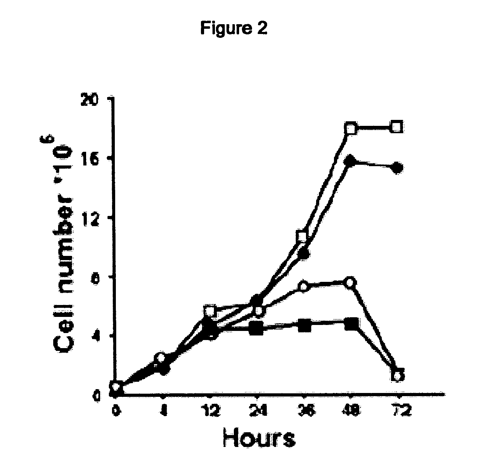

The growth curves of DOHH2 cells in the presence of the various PNAs showed. that PNA***blc-2*** and PNAEµ were equally effective in inhibiting cell growth(Fig.2).

Since the upregulation of the antiapoptotic gene *bcl-2* in FCL cells is responsible for their resistance to apoptosis, we investigated whether PNAEµwt, by inhibiting *bcl-2* hyperexpression, could force apoptotic death of FCL cells.

FCL cells were treated with the different PNAs by addition (stock solution 1 mM in H₂O) directly to the culture medium to a 10 µM final concentration for 12, 36, 72 hours.

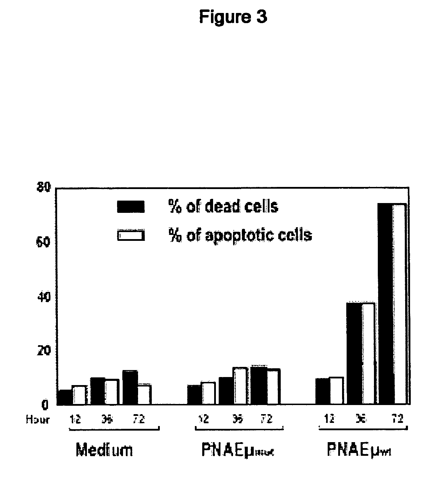

PNA***blc-2*** and PNAEµwt treatment substantially enhanced to a similar level FCL-cell apoptosis. Apoptotic cells and dead cells were detected by Annexin-V binding and PI incorporation, respectively. (Fig.3)

### in vivo treatments

### Animal model (12)

SCID mice (CB-17/IcrHsd, Harlan, Udine, Italy) were maintained at The National Cancer Research Institute's Animal Facility (Sperimentazione su Modelli Animali, IST - Italian Ministry of Health D.M.S.n.44/94-A) in specific pathogen-free conditions. All the mouse studies were carried out following current national regulations regarding the protection of animals used for scientific purpose (D.L.vo 27/01/1992, n. 116), and research protocols were reviewed and approved by an ethics committee. The well-being and health of experimental animals were controlled by a veterinarian to prevent pain and avoidable suffering, distress and lasting harm.

Female SCID mice 10, (5 weeks old, weight about 20 g) were inoculated subcutaneously
with 0.5,1.5, 3.0 x 10⁵ DOHH2 cells in 100 µl of medium.

Only the group of mice inoculated with 1.5x10⁵ cells reproducibly developed tumors and still remained relatively healthy up to 4 weeks after inoculum.

### PNAEµ tumor cure. (13)

In pilot experiments we tested whether PNAEµwt administration to SCID mice after inoculation with FCL cells could block tumor growth. Groups of five female SCID mice per experiment were injected s.c. with DOHH2 cells (as above).

In each experiment one group of mice was left untreated (control). Two other matching mouse groups, 10 days after inoculation, were injected i.v. every other day (100 µl of 1mM PNAs in isotonic saline) with PNAEµwt and PNAEµmut, respectively.

### DOHH2 cells growth in SCID mice

All mice were monitored for weight variation at every injection.

Tumor volume was measured daily to determine the time of appearance of tumors. Tumor size was calculated at the time of every injection from the measurement in mm³ as (V¼ 0.523 x length in mm x width² in mm²) (13). The experiment was terminated, by killing the mice when their pathologic symptoms and discomfort became too severe for any of the treatment groups. Mice were killed in a saturated CO₂ chamber and autopsies were performed.

### Reduction of tumor size by PNAEµwt injection in tumor-bearing mice

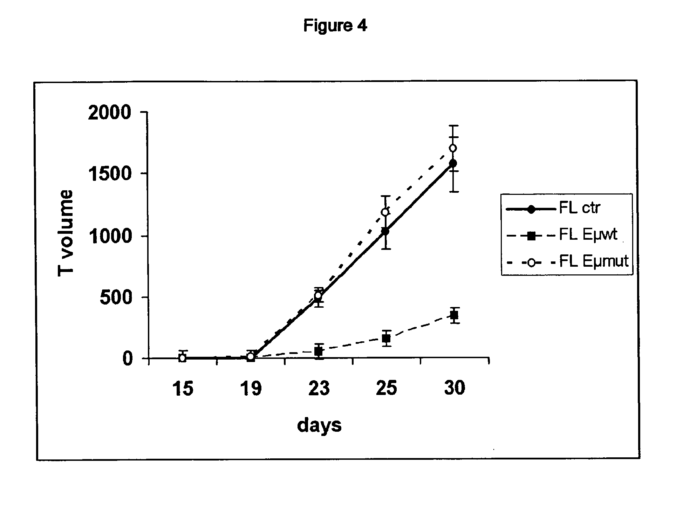

In the two control groups, tumors were detectable at day 16 after inoculum while in the PNAEµwt-treated mouse tumors were palpable at day 20 only. The growth trend of tumors in the two control (1 and 2) sets of mice was very similar. In these mice tumors continued to linearly increase until the end of the experiment (30 days). In contrast, after eight injections of PNAEµwt (around day 26), the tumor volume started to plateau. In PNAEµwt-treated mouse groups, the tumor mass was consistently in the range of a quarter of the controls. These preliminary but significant results are shown in the graphs in Figure 4.

### Tumor characterization

### Tumor cell recovery and characterization. (13)

Subcutaneous tumors, well-defined and nonmetastatic, were isolated and placed quickly in medium under sterile conditions or fixed in formalin.

### Cell surface/cytoplasmic markers. (13)

Tumor cells were mechanically gently resuspended in culture medium, freed from tissue debris and clumps, and stained with the following mAbs (Becton Dickinson, Sunnybay, CA, USA) specific for CD19, CD20, and for marker CD3 as a negative control. These reagents were used in direct immunofluorescence with a goat anti-mouse Ig antibody. In the case of BCL2 molecules, both surface and intracytoplasmic staining of permeabilized cells was carried out. Staining for BCL2 protein was also performed (Clone 8C8; Calbiochem, San Diego, CA, USA) on permeabilized cells and analyzed by flow cytometry using a FACStar^{™} analyzer (Becton Dickinson). Control preparations consisted of unstained cells, cells stained with the (fluorescein isothiocyanate)FITC-labeled second reagent alone, and cells treated with an unrelated Ab followed by the FITC-labeled second reagent.

Cells from all mouse groups expressed CD20, but not CD3, on their surface and also had intracytoplasic BCL2 protein indicating their origin from the inoculated DOHH2-FCL cells. BCL2 cellular level was determined by Western blot analysis as already reported for the *in vitro* treatment in FCL cells from tumors of chronically PNAEµwt and PNAEµmut treated mice.(data not shown) (10, 11)

### Tumor tissue morphological and histological analysis. (13)

Subcutaneous tumor masses were removed from the mice, fixed in formalin and embedded in paraffin. Paraffin-embedded tissues are sectioned at 4 mm, mounted on charged slides, deparaffinized in xylene and rehydrated through graded alcohols. Tissues morphology is evaluated by microscope analysis after hematoxylin and eosin staining. *[In US patent practice if one discloses a protocol that not yet been performed, use present tense.]*

### Toxicity

Injections of the maximum tolerated volume (100 µl) of saturated PNAEµ-NLS (1 mM) solution in isotonic saline, pH 7.0, were well tolerated and, therefore, not toxic. Consequently, an LD₅₀ of the compound could not be evaluated under these experimental conditions.

The invention provides an alternative to the available (rather ineffective and toxic) pharmacologic treatments of FCL.

PNAEµ-NLS is targeted to a regulatory noncoding DNA sequence and is a non-natural molecule. Therefore, its therapeutic use provides the following potential advantages
- long persistence of the intact active molecule, even if administered at pharmacological concentration over a prolonged period of time;
- low or no toxicity;
- low or no immunogenicity;
- low or no mutagenicity.

Since presently the standard FCL treatment consists in high dose conventional chemotherapy, in combination with specific monoclonal antibodies and, in case of failure, autologous stem cell transplantation (ASCT) or radioimmunotherapy (RIT), the invention has tremendous innovative potential.

### References

1. Biagi JJ and. Seymour JF. Insights into the molecular pathogenesis of follicular lymphoma arising from analysis of geographic variation Blood, 2002; 99:4265-4275.
2. Dreyling M, Trümper L., von Schilling C., Rummel M,. Holtkamp U, Waldmann A.,. Wehmeyer J, Freund M. Results of a national consensus workshop: therapeutic algorithm in patients with follicular lymphoma:role of radioimmuno therapy Ann Hematol DOI 10.1007/s00277-006-0207-0.
3. Weigert O, Illidge T, Hiddemann W, Dreyling M, Recommendations for the Use of Yttrium-90 Ibritumomab Tiuxetan in Malignant Lymphoma. Cancer 2006;107:686-95.
4. MR. Smith, Jin F, and Josh I. Enhanced efficacy of therapy with antisense BCL-2oligonucleotides plus anti-CD20 monoclonal antibody in scid mouse/human lymphoma xenografts Mol Cancer Ther 2004;3:1693-9.
5. Tsujimoto Y, Cossman J, Jaffe E, Croce CM: Involvement of the bcl-2 gene in human follicular lymphoma. Science 1985, 228:1440-1443.
6. Aster JC and Longtine JA. Detection of BCL2 Rearrangements in Follicular Lymphoma. American Journal of Pathology, 2002;. 160; 759-763.
7. Cleary ML, Smith SD, Sklar J: Cloning and structural analysis of cDNAs for bcl-2 and a hybrid bcl-2/immunoglobulin transcript resulting from the t(14;18) translocation. Cell 1986, 47:19-28.
8. Graninger WB, Seto M, Boutain B, Goldman P, Korsmeyer SJ: Expression of Bcl-2 and Bcl-2-Ig fusion transcripts in normal and neoplastic cells. J Clin Invest 1987, 80:1512-1515.
9. Kuppers, R., and Dalla Favera, R. Mechanisms of chromosomal translocations in B-cell lymphomas. Oncogene, 20: 5580-5594, 2001.
10. Cutrona G, Carpaneto EM, Ulivi M, Roncella S, Landt O, Ferrarini M et al. Effects in live cells of a c-myc anti-gene PNA linked to a nuclear localization signal. Nat Biotech 2000; 18: 300-303.
11. Cutrona G, Carpaneto EM, Ponzanelli A, Ulivi M, Millo E, Scarf S et al. Inhibition of the translocated c-myc in Burkitt's lymphoma by a PNA complementary to the Em enhancer. Cancer Res 2003; 63: 6144-6148.
12. Boffa LC, Cutrona G, Cilli M, Mariani MR, Matis S, Pastorino M et al. Therapeutically promising PNA complementary to a regulatory sequence for c-myc: pharmacokinetics in an animal model of human Burkitt's Lymphoma. Oligonucleotides 2005; 15: 85-93.
13. Boffa, L.C., Cutrona, G., Cilli, M., Matis, S., Damonte, G., Mariani, M.R., Millo, E., Moroni, M., Roncella, S., Fedeli, F. and Ferrarini, M. (2007). Inhibition of Burkitt's lymphoma cells growth in SCID mice by a PNA specific for a regulatory sequence of the translocated c-myc. Cancer Gene Therapy 14:220-226.
14. Nielsen PE.Peptide nucleic acid targeting of double-stranded DNA. Methods Enzymol 2001; 340: 329-340.
15. Boffa, L.C., Menichini, P., Bolognesi, C., Cutrona, G., Roncella, S., Damonte, G., Millo, E.,. Mariani, M.R, Matis, S., Russo, D., Ciliutti, P., and M. Ferrarini(2007). Lack of mutagenicity and clastogenicity of a PNA targeted to a regulatory sequence of the translocated c-myc oncogene in Burkitt's Lymphoma. Mut. Res. Genet. Toxicol. Environ. Mutagen. 628: 129-137.
16. Cutrona G,. Boffa LC, Mariani MR, Matis S, Damonte G, Millo E, Roncella S,and Ferrarini M.(2007) The PNA targeted to a regulatory sequence of the translocated c-myc oncogene in Burkitt's Lymphoma lacks immunogenicity Follow up characterization of PNAEµ-NLS. Oligonucleotides. 171:146-150.
17. Kluin-Nelemans HC, Limpens J, Meerabux J, Beverstock GC, Jansen JH, de Jong D, Kluin PM. Leukemia. 1991 3:221-4.

## Claims

1. The use of Peptide Nucleic Acids complementary to the Eµ enhancer of the IgH chain locus on chromosome 14 (PNAEµwt) for the preparation of a medicament for the treatment of Follicular Cell Lymphoma.

2. The use according to claim 1 wherein the PNAEµwt and its negative and positive controls are selected from PNA oligomers having the following sequences.
- GCAGGAAGCAGGTCACCG-NLS PNAEµwt
- GTAGGAAGTAGGTCATCG-NLS. PNAEµmut
- CTACGAGTGGGATGCG-NLS. PNA*bcl-2*

3. A Peptide Nucleic Acid having the sequence CTACGAGTGGGATGCG-NLS.
